# FASCICULE DE BREVET EUROPEEN

(11) **EP 4 198 483 B1**
(45) Date de publication et mention de la délivrance du brevet: **03.07.2024**
(21) Numéro de dépôt: 22211239.3
(22) Date de dépôt: 02.12.2022
(51) Int. Cl.: G01N 1/22, B01L 3/00

(54) **PROCÉDÉ ET DISPOSITIF DE RÉCUPÉRATION ET D'ANALYSE DE PARTICULES AÉROPORTÉES**
VERFAHREN UND VORRICHTUNG ZUR RÜCKGEWINNUNG UND ANALYSE VON LUFTGETRAGENEN PARTIKELN
AIRBORNE PARTICLE RECOVERY AND ANALYSIS METHOD AND DEVICE

(30) Priorité: 17.12.2021 FR 2113752
(43) Date de publication de la demande: 21.06.2023
(73) Titulaire: Commissariat à l'énergie atomique et aux énergies alternatives, 75015 Paris (FR)
(72) Inventeur: BLAIRE, Guillaume, 38054 Grenoble cedex 09 (FR); ALESSIO, Manuel, 38054 Grenoble cedex 09 (FR); BAQUE, Mélissa, 38054 Grenoble cedex 09 (FR); ROUX, Jean-Maxime, 38054 Grenoble cedex 09 (FR)
(74) Mandataire: INNOV-GROUP

(56) Documents cités:
- WO-A2-2005/089108
- US-A1- 2012 174 650
- US-B1- 10 161 835

## Description

### Domaine technique de l'invention

La présente invention se rapporte à un procédé et à un dispositif de récupération et d'analyse de particules aéroportées.

### Etat de la technique

Plusieurs solutions ont déjà été proposées dans l'état de la technique pour collecter des particules présentes dans un aérosol, dans un but d'analyse de ces particules.

Une méthode de séparation connue et particulièrement avantageuse est de type électrostatique. Elle est mise en oeuvre dans des collecteurs électrostatiques, aussi appelés électrofiltres ou filtres électrostatiques ou encore précipitateurs électrostatiques (ESP pour *"electrostaticprecipitator*" en anglais).
Il existe plusieurs catégories de collecteurs électrostatiques, parmi lesquelles :
- Les collecteurs électrostatiques dits secs, par exemple décrits dans la demande de brevet WO2015/197747A1**,**
- Les collecteurs électrostatiques dits humides, par exemple décrits dans la demande de brevet WO2004/041440A1**,**
- Les collecteurs électrostatiques dits semi-humides, par exemple décrits dans WO2007/012447A1**.**

Dans toutes ces catégories, les collecteurs comportent une chambre dans laquelle est injecté ou aspiré un flux d'air contenant les particules et génèrent un champ électrique entre deux électrodes, une électrode de décharge et une contre-électrode dite de collecte, qui est en règle générale reliée à la masse.

Le champ électrique créé entre les deux électrodes génère un flux d'ions à partir d'une poche de gaz ionisée entourant l'électrode de décharge. Le flux d'air contenant les particules est injecté à travers le flux d'ions. En présence d'ions, les particules acquièrent des charges électriques et deviennent ainsi sensibles au champ électrique généré entre les deux électrodes et sont entraînées par la force électrique vers la contre-électrode. Dans la première catégorie des collecteurs électrostatiques dits secs, les particules collectées sont décrochées des électrodes de collecte par un procédé sec, par exemple en mettant les électrodes en vibration ou encore par les frottements mécaniques de brosses sur leur surface.

Dans la deuxième catégorie des collecteurs électrostatiques dits humides, les particules capturées sur l'électrode de collecte sont évacuées par ruissellement d'eau sur celle-ci. Dans la troisième catégorie des collecteurs électrostatiques dits semi-humides, de la vapeur d'eau est introduite dans la chambre contenant l'électrode de décharge ou en amont de celle-ci. Les particules en suspension dans l'air croissent alors par nucléation hétérogène pour former des gouttelettes et lesdites gouttelettes sont précipitées sur la contre-électrode par la force électrique. La vapeur introduite peut en outre condenser sur les parois et ainsi conduire à un ruissellement sur l'électrode de collecte qui contribue à l'évacuation des particules capturées.

La publication référencée *"*Hyeong Rae Kim, Sanggwon An, and Jungho Hwang, Aerosol-to-Hydrosol Sampling and Simultaneous Enrichment of Airborne Bacteria For Rapid Biosensing, ACS Sens. 2020, 5, 2763-2771*"* présente un exemple récent de collecteur électrostatique destiné à collecter des bio-particules dans un flux d'air. Le flux d'air est injecté dans un canal et passe entre deux électrodes. Les particules en suspension dans l'air sont attirées vers l'électrode de collecte par précipitation électrostatique. Un liquide est injecté en continu pour éluer les particules capturées sur l'électrode de collecte. Cette solution nécessite notamment un flux de liquide continu pour éluer les particules présentes sur l'électrode de collecte, ce qui rend la solution peu adaptée pour être facilement mise en oeuvre dans un instrument porté ou déployé par exemple, avec un drone. Il existe en effet, en fonctionnement, un risque de voir le liquide d'élution se disperser et s'écouler dans le canal d'air si le dispositif est penché. Le liquide pourrait également former un pont entre les électrodes si le dispositif était renversé et un court-circuit entre les deux électrodes se formerait. Le même problème est posé par les solutions techniques décrites dans les brevets WO2004/041440A1 et WO2007/012447A1 précités.

Un autre problème présenté par les solutions existantes est la réutilisation du même dispositif de collecte. Si des pathogènes sont collectés lors d'un prélèvement il est nécessaire de décontaminer toutes les surfaces sur lesquelles ils se sont déposés. Or le canal dans lequel circule l'air, les électrodes et des tubulures mises en oeuvre pour apporter le liquide d'élution et pour l'entrainer vers une chambre de récupération sont difficiles d'accès. Il serait avantageux de disposer d'une solution technique qui permette d'enchainer les prélèvements sans devoir décontaminer le dispositif de collecte et d'élution.

De manière générale, les particules collectées et éluées sont extraites du collecteur pour être analysées dans une chambre externe. La chambre externe possède toutes les caractéristiques permettant de mettre en oeuvre une analyse fiable de ces particules, par exemple par amplification biomoléculaire (PCR, LAMP ou autres). Un opérateur est souvent chargé de ce transfert ainsi que de faire en sorte que les conditions de mise en oeuvre de la réaction, notamment au niveau de la température, sont bien suivies.

Il n'existe pas de solutions totalement intégrées et quasiment autonomes dans lesquelles les particules sont automatiquement envoyées vers une chambre pour être analysées, ou alors les solutions existantes sont complexes. A titre d'exemple, la publication référencée *"*X. Jiang, J.C. Loeb, M. Pan et al., Intégration of sample préparation with RNA-Amplification in a handheld device for airborne virus détection, Analytica Chimica Acta 1165 (2021) 338542" décrit un dispositif et un procédé permettant de collecter des particules en suspension dans l'air, apporter des réactifs et détecter par amplification biomoléculaire la présence de pathogènes. Mais le dispositif comporte de très nombreux composants qui doivent être montés avant emploi puis, entre deux prélèvements et analyses, qui doivent être démontés et notamment séparés du dispositif de collecte pour être remplacés. L'ajout des réactifs pour les analyses comporte également une étape manuelle de rotation qui pourrait certes être automatisée mais au moyen d'un moteur et des articulations mécaniques qui ajoute encore de la complexité au dispositif.

Les documents US2012/174650A1**,** WO2005/089108A2 et US10,161,835B1 décrivent des solutions de récupération et d'analyse de particules aéroportées.

Le but de l'invention est de proposer un procédé et un dispositif de collecte, de récupération et d'analyse de particules aéroportées, dans lequel la récupération et l'analyse des particules aéroportées peuvent être mise en oeuvre de manière simple et fiable, sans risque de disperser le liquide d'élution en fonctionnement, sans utiliser des moyens complexes telles que des articulations mécaniques pour assurer les étapes de préparation d'échantillons et d'analyses, et sans risque de transferts de contaminations d'un prélèvement à un autre.

### Exposé de l'invention

Ce but est atteint par un procédé de récupération et d'analyse de particules aéroportées, comprenant :
- Une étape d'élution de particules collectées sur une surface de collecte, par injection d'un liquide d'élution à travers un circuit fluidique d'élution, ledit circuit fluidique d'élution étant agencé pour acheminer lesdites particules collectées à destination d'une chambre de réaction d'un module d'analyse,
- Une étape d'analyse des particules aéroportées récupérées dans la chambre de réaction,
- L'étape d'élution étant mise en oeuvre par chauffage d'un premier réservoir contenant ledit liquide d'élution à une première valeur de température pour faire fondre un premier composé fusible maintenant ledit premier réservoir sous pression,
- L'étape d'analyse étant mise en oeuvre par chauffage de la chambre de réaction à une deuxième valeur de température, supérieure à la première valeur de température, pour :
   ∘ Activer une réaction de détection dans ladite chambre de réaction en vue de réaliser une analyse des particules aéroportées,
   ∘ Isoler ladite chambre de réaction pendant la réaction de détection par activation d'un dispositif d'isolation de la chambre de réaction,
   ∘ Amorcer un dispositif de scellement de la chambre de réaction, par fusion d'un deuxième composé fusible,
- Une étape de scellement de ladite chambre de réaction par figeage du deuxième composé fusible lorsque la température descend à une troisième valeur inférieure à ladite deuxième valeur.

Selon une particularité, le procédé comporte une étape de contrôle de la réaction par injection d'un deuxième liquide d'élution dans au moins une chambre de contrôle identique à ladite chambre de réaction.

Selon une autre particularité, chaque liquide d'élution embarque des réactifs utilisés lors de la réaction dans la chambre de réaction.

Selon une autre particularité, la réaction est de type amplification biomoléculaire. Selon une autre particularité, l'activation du dispositif d'isolation de la chambre de réaction consiste à fermer une première vanne d'isolation agencée sur le circuit fluidique d'élution et dotée d'une membrane déformable.

Selon une réalisation particulière, l'amorçage du dispositif de scellement de la chambre de réaction consiste à libérer le deuxième composé fusible dans le circuit fluidique d'élution.

Selon une autre réalisation particulière, l'amorçage du dispositif de scellement de la chambre de réaction consiste à libérer le deuxième composé fusible pour que celui-ci vienne se déposer sur la membrane déformable de la première vanne d'isolation.

L'invention concerne également un dispositif de récupération et d'analyse de particules aéroportées adapté pour mettre en oeuvre le procédé tel que défini ci-dessus, le dispositif comprenant :
- Un module d'élution de particules collectées sur une surface de collecte, par injection d'un liquide d'élution à travers un circuit fluidique d'élution, ledit circuit fluidique d'élution étant agencé pour acheminer lesdites particules collectées à destination d'une chambre de réaction d'un module d'analyse,
- Un module d'analyse des particules aéroportées récupérées dans la chambre de réaction,
- Le module d'élution comportant un premier réservoir dans lequel est injecté le liquide d'élution maintenu sous pression par un premier composé fusible, ledit premier composé fusible étant choisi pour fondre par un premier chauffage à une première valeur de température en vue de libérer ledit liquide d'élution,
- Le module d'analyse comportant :
   ∘ Un dispositif d'isolation de la chambre de réaction, configuré pour isoler ladite chambre de réaction lors d'un deuxième chauffage de la chambre de réaction à une deuxième valeur de température supérieure à ladite première valeur de température,
   ∘ Un dispositif de scellement de la chambre de réaction, amorcé par fusion d'un deuxième composé fusible configuré pour figer à une troisième valeur inférieure à ladite deuxième valeur.

Selon une particularité, le dispositif comporte un module de contrôle de la réaction comportant au moins une chambre de contrôle identique à ladite chambre de réaction dans laquelle est injecté un deuxième liquide d'élution.

Selon une autre particularité, chaque liquide d'élution embarque des réactifs utilisés lors de la réaction dans la chambre de réaction.

Selon une autre particularité, la réaction est de type amplification biomoléculaire. Selon une autre particularité, le dispositif d'isolation de la chambre de réaction comporte une première vanne d'isolation agencée sur le circuit fluidique d'élution et dotée d'une membrane déformable.

Selon une réalisation particulière, le dispositif de scellement de la chambre de réaction comporte un corps du deuxième composé fusible positionné pour se libérer dans le circuit fluidique d'élution lors de l'amorçage.

Selon une autre réalisation particulière, le dispositif de scellement de la chambre de réaction comporte un corps du deuxième composé fusible positionné pour se déposer sur la membrane déformable lors de l'amorçage.

Selon une particularité, le dispositif comporte un composant de collecte et d'analyse de particules aéroportées réalisé sous la forme d'un élément monobloc, comportant ;
- Un module de collecte des particules aéroportées ;
- Le module d'élution des particules aéroportées collectées ;
- Le module d'analyse des particules éluées vers la chambre de réaction ;

Selon une autre particularité, le dispositif comporte un module de chauffage réalisé sous la forme d'un instrument sur lequel ledit composant de collecte et d'analyse vient s'adapter de manière amovible.

### Brève description des figures

D'autres caractéristiques et avantages vont apparaître dans la description détaillée qui suit faite en regard des dessins annexés dans lesquels :
- La figure 1 représente de manière schématique un composant de collecte et d'analyse de particules aéroportées, conforme à l'invention et adapté pour mettre en oeuvre le procédé de l'invention.
- La figure 2 représente de manière schématique le principe de réalisation du module d'élution utilisé dans le composant de l'invention ;
- La figure 3 illustre, par des vues en coupe, le principe de fonctionnement du module d'élution de la figure 2 ;
- La figure 4 représente de manière schématique le principe de réalisation du dispositif d'isolation employé dans le composant de l'invention ;
- La figure 5 illustre, par des vues en coupe, le principe de fonctionnement du dispositif d'isolation de la figure 4 ;
- La figure 6A illustre, de manière schématique, le principe de fonctionnement du dispositif de scellement utilisé dans le composant de l'invention ;
- La figure 6B illustre, de manière schématique, le principe de fonctionnement d'une variante de réalisation du dispositif de scellement utilisé dans le composant de l'invention ;
- Les figures 7A à 7F illustrent les différentes étapes du procédé conforme à l'invention ;
- La figure 8 montre plusieurs diagrammes illustrant le cyclage en température en corrélation avec l'état pris par chaque vanne employée dans le composant de l'invention ;
- La figure 9 montre de manière schématique une variante de réalisation avantageuse du composant de l'invention ;

### Description détaillée d'au moins un mode de réalisation

Dans la suite de la description, les termes "amont" et "aval" sont à comprendre en tenant compte du sens de circulation du fluide dans le circuit fluidique considéré.

Dans la suite de la description, une vanne à l'état ouvert laisse passer le fluide (état 1 ou ON) et une vanne à l'état fermé bloque le passage du fluide (état 0 ou OFF).

Un système complet permettant la collecte de particules aéroportées, la récupération des particules collectées et l'analyse des particules comportant plusieurs modules fonctionnels :
- Un module M1 de collecte des particules aéroportées ;
- Un module M2 fluidique d'élution des particules aéroportées collectées ;
- Un module M3 d'analyse des particules éluées vers une chambre de réaction ;
- Un module M4 de chauffage, commun à plusieurs desdits autres modules ;
- Un module M5 de contrôle configuré pour commander les différents modules fonctionnels du composant ;

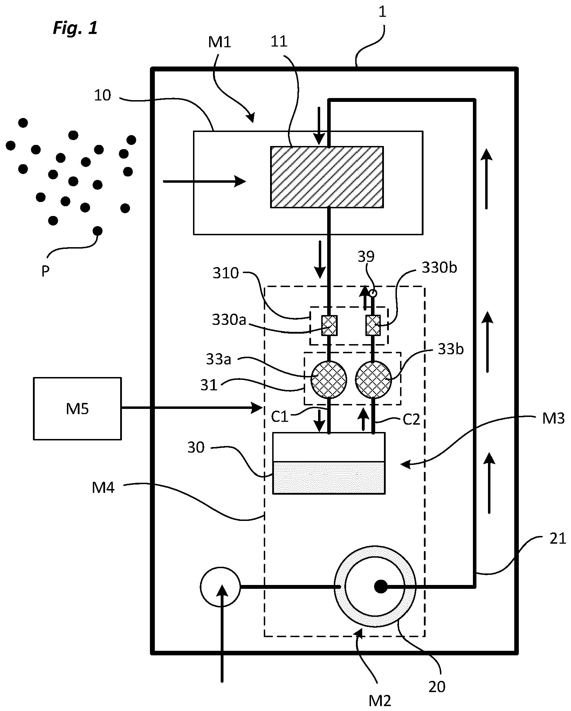

La figure 1 représente un composant 1 de collecte et d'analyse capable de mettre en oeuvre certaines étapes d'un procédé de collecte et d'analyse dans ce composant.

Le composant 1 de collecte et d'analyse se présente avantageusement sous la forme d'un élément monobloc, regroupant par exemple plusieurs des modules fonctionnels évoqués ci-dessus, notamment :
- Le module M1 de collecte des particules aéroportées ;
- Le module M2 fluidique d'élution des particules aéroportées collectées ;
- Le module M3 d'analyse des particules éluées vers une chambre de réaction ;

Le module M4 de chauffage est commun à plusieurs desdits autres modules et fait avantageusement partie d'un instrument sur lequel le composant peut venir s'adapter. Sur les figures annexées, le module M4 est représenté par une zone en pointillés, correspondant à sa zone d'influence sur les modules du composant 1.

De même, le module M5 de contrôle, configuré pour commander les différents modules fonctionnels du composant en vue de collecter les particules P et d'analyser les particules, fait avantageusement partie de l'instrument évoqué ci-dessus.

Dans le but de réaliser une balise entièrement autonome, le module de chauffage M4 peut cependant être intégré au composant ou assemblé sur celui-ci. Il en est de même pour le module de contrôle M5. Dans ce cas, le système pourra également comporter une source d'alimentation électrique telle qu'une batterie embarquée.

Par le terme "module" il faut comprendre un ou plusieurs éléments ou composants matériels et éventuellement logiciels permettant de mettre en oeuvre une ou plusieurs étapes du procédé de l'invention.

Les particules P peuvent être des micro-particules ou des nano-particules, biologiques ou non, présentes en suspension dans l'air sous forme d'un aérosol.

De manière non limitative, la collecte des particules P peut notamment être réalisée dans l'air ambiant ou dans l'air expiré par un être vivant. Dans la suite de la description, on considérera que la collecte des particules est réalisée dans l'air ambiant.

L'un des objectifs est d'analyser les particules P en vue de détecter la présence d'un agent pathogène ou une trace de sa présence.

La solution peut présenter les particularités suivantes :
- Les agents pathogènes recherchés peuvent être, entre autres, des microorganismes tels que des virus, des bactéries, des spores fongiques, des toxines, des mycotoxines, des allergènes, ou tout autre agent nuisible.
- Les particules P sont avantageusement collectées dans un but d'analyse. L'analyse peut consister à détecter la présence d'ADN, d'ARN, de protéines, d'éléments composants l'agent pathogène, tels que lipides ou glucides, d'un ou plusieurs agents pathogènes présents dans les particules collectées. L'analyse peut également consister à détecter des molécules telles que l'ATP ou encore des sucres tels que le mannitol, l'arabitol et le glucose qui renseignent sur la présence de microorganismes. L'analyse peut aussi porter sur la détection de molécules telles que des allergènes et des mycotoxines.
- A titre d'exemple, la méthode d'analyse peut être de type amplification biomoléculaire (par exemple de type LAMP, RPA, PCR,...), de type immuno-enzymatique (par exemple de type ELISA) ou immuno-chromatographique (par exemple de type bandelette).
- Le composant 1 pourra notamment être employé sous la forme d'une balise de surveillance. Pour cela, il devra alors présenter une certaine autonomie de fonctionnement, c'est-à-dire pouvoir collecter et analyser les particules, avec un minimum d'intervention extérieure.

Le module M1 de collecte fonctionne avantageusement par effet électrostatique. De manière non limitative, il peut comporter deux électrodes, une électrode de décharge reliée à un potentiel électrique et une contre-électrode dite de collecte, qui est en règle générale reliée à la masse. Les deux électrodes sont éloignées l'une de l'autre de manière à créer un champ électrostatique suffisant pour attirer les particules P aéroportées vers l'électrode de collecte, celles-ci étant destinée à être captées et piégées au niveau d'une surface de collecte associée à cette électrode de collecte.

Le module de collecte peut notamment comporter un canal 10 de collecte dans lequel est injecté ou aspiré un flux d'air contenant les particules. Des moyens de collecte générateurs du flux d'air sont par exemple configurés pour diriger les particules P présentes dans l'air vers l'électrode de collecte contenu dans ledit canal 10 de collecte. La circulation de l'air à travers le canal 10 peut être forcée (par exemple à l'aide d'un ventilateur) ou non. Les particules P peuvent être collectées sur une membrane 11, formant la surface de collecte et pouvant intégrer l'électrode de collecte.

Le composant 1 peut également intégrer un module M2 fluidique d'élution (ci-après module d'élution) comportant au moins un circuit d'élution. Ce circuit d'élution comporte un réservoir 20 d'éluant et un canal fluidique d'élution 21 débouchant d'un côté dans le réservoir et de l'autre côté vers la surface de collecte. Il est utilisé pour acheminer le liquide d'élution jusqu'à la surface de collecte et permet de déverser le liquide d'élution sur la surface de collecte pour détacher les particules P et les transporter vers le module M3 d'analyse.

Le réservoir 20 d'éluant contient avantageusement le liquide d'élution, par exemple un liquide tel que l'eau, ce fluide permettant la mise en suspension des particules collectées au niveau de la surface de collecte. Le liquide d'élution peut aussi embarquer au moins une partie des réactifs nécessaires à la mise en oeuvre de la réaction de détection au niveau de la chambre d'analyse.

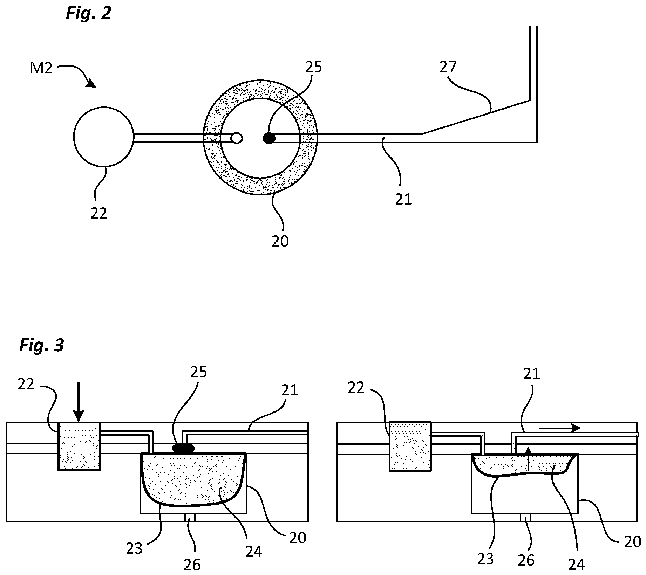

Comme représenté sur la figure 2 et la figure 3, le module M2 d'élution peut comporter un port d'entrée 22 bloqué par un septum, par lequel est injecté le liquide d'élution 24 vers son réservoir 20. Le module M2 d'élution peut comporter une membrane 23 déformable logée dans le réservoir 20 et apte à se déformer vers l'intérieur du réservoir 20 sous la pression du liquide d'élution 24 injecté par le port d'entrée 22. Le réservoir 20 est initialement scellé par un dépôt d'un premier composé fusible 25, formant un bouchon, permettant ainsi sa mise sous pression lors de l'injection du fluide 24. Sous la membrane 23, le module d'élution comporte avantageusement un évent 26, prévu pour chasser l'air présent, lors de la mise sous pression du réservoir 20. La présence de l'évent 26 permet notamment d'obtenir un volume calibré et reproductible dans le réservoir 20.

Le premier composé fusible 25 peut être déposé sous la forme d'une bille, par exemple à l'aide d'une micropipette, afin d'isoler le réservoir du canal fluidique d'élution. Cette bille est déposée sous forme liquide. Pour industrialiser ce processus, on pourrait imaginer l'utilisation d'un robot pipeteur, ou le dépôt du premier matériau fusible sous forme solide suivi par une chauffe locale. En refroidissant cette bille du composé fusible ferme de manière étanche la communication entre la chambre et le canal fluidique d'élution. Un joint, de type silicone ou autre peut être inséré entre la sortie du réservoir 20 et le composé fusible, ceci afin de compenser les éventuelles différences de dilation thermique entre le matériau fusible et le matériau entourant le via d'accès au réservoir 20.

En sortie du réservoir, le module d'élution peut comporter une zone de piégeage 27 du composé fusible. Cette zone se matérialise par une partie évasée du canal 21 fluidique d'élution. Le volume de composé fusible utilisé doit être inférieur au volume de la zone de piégeage du matériau.

Le composé fusible peut être une paraffine, capable de fondre à une première température. On dépose par exemple 2µl de paraffine pour un volume de la zone de piégeage de 4µl.

Le principe de fonctionnement du module d'élution est le suivant :
- Le réservoir 20 étant scellé par le bouchon réalisé par le premier composé fusible 25, on injecte le liquide d'élution 24 à travers le septum 22. Cette injection de fluide 24 met sous pression le réservoir 20 en déformant la membrane 23. Sous cette membrane en silicone, l'évent 26 permet de chasser l'air présent avant remplissage. Typiquement, un volume de 100µl est injecté dans le réservoir 20. La membrane 23, très déformable, vient épouser la forme du réservoir 20, permettant ainsi de remplir ce réservoir 20 avec un volume calibré et reproductible.
- Le module M5 de contrôle présent dans l'instrument qui met en oeuvre le composant 1 active le module M4 de chauffage de l'instrument pour chauffer le premier composé fusible 25 présent dans le composant 1 au-dessus de sa température de fusion. Par exemple, si le composé fusible est une paraffine telle que le docosane (température de fusion=45°C), on chauffera jusqu'à atteindre une température de 48°C.
- Dès que le premier composé fusible 25 fond, il s'écoule et se trouve poussé par la pression présente dans le réservoir 20 et dans le canal fluidique d'élution 21. La zone de piégeage 27, qui est toujours à basse température car en dehors de la zone d'influence du module M4 de chauffage, permet, avec sa forme évasée, de ralentir à cet endroit la vitesse du fluide et d'offrir une zone ayant un volume suffisamment important pour stocker le premier composé fusible qui s'y solidifie. Ce dernier, en refroidissant, se fige préférentiellement sur les parois du canal fluidique d'élution 21 au niveau de sa partie évasée, tout en laissant un passage suffisant pour permettre au liquide d'élution 24 de s'écouler à travers le canal fluidique d'élution 21. La pression est maintenue dans le canal par la membrane 23 et permet au fluide d'atteindre le module M1 de collecte, sans autre moyen.

Dans une variante de réalisation, l'évent 26 peut être connecté à un second réservoir clos. Ce réservoir est mis sous pression lorsque la membrane 23 est déformée par le fluide. La membrane 23 peut être complètement déformée, garantissant le volume puisque l'air sera comprimé dans ce second réservoir. On peut ainsi augmenter la pression dans le réservoir 20, faire en sorte que cette pression soit calibrée (par la taille du second réservoir) et obtenir un volume de liquide calibré.

Comme indiqué ci-dessus, le module M3 d'analyse des particules P collectées est avantageusement également implanté dans le composant 1, afin de réduire le matériel qu'un opérateur devrait manipuler et ainsi automatiser le procédé.

Le module M3 d'analyse comporte principalement une chambre 30 de réaction dans laquelle débouche le canal fluidique d'élution 21 et avantageusement un canal débouchant vers l'extérieur pour former un évent 39.

La chambre 30 de réaction est avantageusement réalisée dans le composant. Cette chambre 30 de réaction peut embarquer au moins une partie des réactifs nécessaires à l'analyse, par exemple pour mettre en oeuvre une réaction d'amplification ou équivalent. Comme évoqué précédemment, l'analyse peut être réalisée par amplification biomoléculaire ou être de type immuno-enzymatique (type ELISA).

Une analyse par amplification biomoléculaire de microorganismes suppose une extraction du matériel génomique des microorganismes. Différentes solutions techniques peuvent bien entendu être mises en oeuvre pour cela. Avantageusement la lyse des microorganismes est réalisée par voie thermique, ainsi le module de chauffage M4 du système sert à la fois à l'élution des microorganismes collectés, éventuellement à la lyse qui conduit à l'extraction du matériel génomique et à l'amplification biomoléculaire. A titre d'exemple une étape de chauffage à 65°C de la chambre 30 permet d'extraire le matériel génétique de certains virus.

Dans certaines conditions, il est nécessaire d'isoler ou même de sceller la chambre 30, que ce soit lors de la réaction de détection, pour éviter l'évaporation de la solution pendant l'étape de chauffage nécessaire à l'amplification biomoléculaire, ou pour éviter de polluer le mélange, ou encore pour assurer le transport du composant 1 de manière sûre.

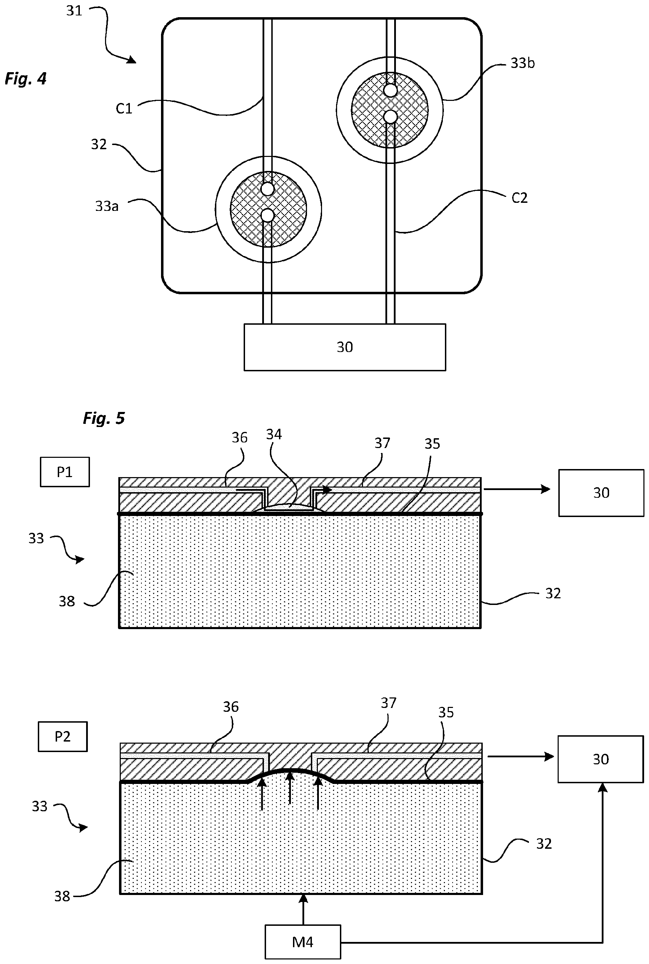

Selon l'invention, en référence à la figure 4 et à la figure 5, le module M3 d'analyse comporte un dispositif 31 d'isolation de la chambre 30 de réaction.

Ce dispositif 31 d'isolation est avantageusement commun aux deux circuits fluidiques débouchant dans la chambre 30 de réaction, c'est-à-dire le circuit fluidique d'élution C1 et le circuit fluidique C2 comportant l'évent 39.

Il comporte au moins un réservoir 32 destiné à contenir un volume d'air 38 et une vanne 33a, 33b (référence 33 de manière générale) d'isolation distincte pour chaque circuit fluidique. Le réservoir 32 d'air est choisi commun aux deux vannes 33a, 33b mais l'utilisation de deux réservoirs distincts pourrait être envisagée.

En référence à la figure 5, chaque vanne 33 d'isolation comporte un espace 34 dans lequel débouche un canal d'entrée 36 et duquel ressort un canal de sortie 37 menant à la chambre 30 de réaction, le volume de l'espace 34 étant variable selon la position d'une membrane 35 déformable. La membrane 35 est apte à se déformer entre une première position dite d'ouverture dans laquelle elle laisse passer le fluide dans le circuit contrôlé (figure 5 - P1) et une position dite de fermeture dans laquelle elle bloque le passage du fluide dans le circuit contrôlé (figure 5 - P2). Dans sa position de fermeture, le volume de l'espace 34 est nul ou quasi-nul, la membrane 35 étant plaquée contre une surface sur laquelle débouche les deux canaux. Selon sa position, la membrane 35 permet donc de moduler le volume de l'espace 34 de la vanne 33 d'isolation.

Pour déplacer la membrane 35 entre sa première position et sa deuxième position, on utilise le module M4 de chauffage. Le module M4 de chauffage est agencé et configuré pour chauffer le volume d'air 38 placé dans le réservoir 32 afin de venir dilater ce volume d'air. En se dilatant dans le réservoir 32, l'air pousse la membrane 35, la déformant vers sa deuxième position de fermeture (P2). La membrane 35 vient alors obturer les deux canaux 36, 37 pour fermer le circuit fluidique.

Il faut noter que le réservoir 32 est fermé de manière étanche dans le composant.

De manière avantageuse, on utilise l'activation du module M4 de chauffage, nécessaire pour la mise en oeuvre de la réaction de détection dans la chambre 30 de réaction pour actionner les membranes 35 des deux vannes 33a, 33b vers leur position de fermeture et ainsi isoler la chambre 30 en fermant les deux circuits fluidiques. Autrement dit, par une seule commande du module M4 de chauffage, on obtient à la fois une isolation à chaud de la chambre 30 de réaction à l'aide des deux vannes 33a, 33b, et la mise en oeuvre de la réaction de détection. A titre d'exemple cette réaction a lieu à 65°C pour l'amplification biomoléculaire isotherme LAMP. Le matériau et les caractéristiques géométriques de la membrane 35 sont alors choisis de façon à obtenir une fermeture des vannes à 65°C.

De manière plus concrète, le principe de fonctionnement du dispositif 31 est le suivant :
- A température ambiante, la chambre 30, la chambre 32 et les deux circuits fluidiques sont à des pressions similaires. La membrane 35 est dans sa position d'ouverture (P1), et le liquide d'élution peut ainsi passer librement.
- Lorsqu'on active le module de chauffage M4, l'air contenu à l'intérieur du réservoir 32 se dilate. En première approximation, l'augmentation de pression est directement proportionnelle à la hausse de température (en K). Ainsi en passant de 25°C à 65°C (298K à 338K) la pression augmente de 13% (autour de 100 mbar) ce qui déforme fortement la membrane 35 de chaque vanne 33.
- Pour chaque vanne 33, la membrane 35 déformée bloque alors les deux canaux du circuit fluidique, isolant la chambre 30 de réaction par rapport à l'extérieur (P2).

Un avantage très intéressant de ce dispositif est d'interdire une éventuelle dilatation des bulles d'air pouvant se trouver dans la chambre 30 de réaction. En effet, une bulle dans la chambre 30 de réaction verra la même hausse de pression que la membrane 35, car elle verra également la même hausse de température. Avec un tel dispositif, la taille de la bulle dans la chambre ne pourra donc pas varier lors de la chauffe.

Ce dispositif, en fermant les deux circuits fluidiques menant à la chambre 30 de réaction, permet aussi de limiter grandement l'évaporation. Ainsi des analyses de 30 min peuvent être menées sans perte de liquide.

Lorsque la température redescend, les pressions entre le réservoir 32 et les circuits fluidiques s'équilibrent et la membrane 35 retrouve sa position d'ouverture d'origine (P1).

Selon un autre aspect de l'invention, on utilise également un dispositif de scellement à froid de la chambre 30, une fois que la réaction de détection est terminée.

Dans le contexte d'une réaction de détection de type amplification biomoléculaire, il est indispensable de pouvoir isoler le volume réactionnel après analyse, ceci afin d'éviter toute contamination, en scellant la chambre 30 de réaction. En effet, si des molécules d'ARN/ADN sont présentes dans l'échantillon à analyser, leur quantité sera amplifiée de plusieurs ordres de grandeur par la réaction. Le nombre d'amplicons après analyse peut donc être très important. Il faut absolument éviter toute fuite de liquide contenant les amplicons au risque de voir l'instrument et son environnement irrémédiablement pollué.

On présente ci-dessous deux variantes de réalisation de ce dispositif de scellement.

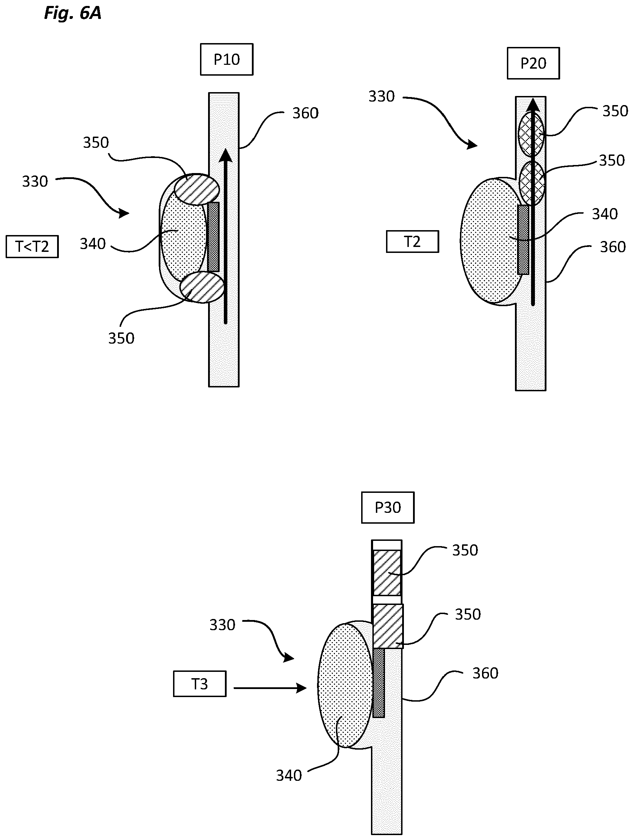

Dans une première variante de réalisation illustrée par la figure 6A, le dispositif 310 de scellement comporte deux vannes 330a, 330b (référence 330 de manière générale) de scellement à froid intégrées au composant.

Sur le circuit fluidique d'élution C1, la première vanne 330a de scellement à froid est positionnée en amont de la première vanne 33a d'isolation à chaud décrite ci-dessus (voir figure 1).

Sur le circuit fluidique C2 comportant l'évent 39, la deuxième vanne 330b de scellement à froid est positionnée en aval par rapport à la deuxième vanne 33b d'isolation à chaud décrite ci-dessus (voir figure 1).

Il faut noter que la deuxième vanne de scellement 330b, placée sur le circuit fluidique C2 comportant l'évent 39, n'est pas indispensable, l'évent 39 pourrait être muni d'une membrane barrière hydrophobe.

En référence à la figure 6A, chaque vanne 330 est composée d'au moins deux corps 350 d'un deuxième composé fusible, piégeant initialement un élément capable de se dilater, dans le canal 360 du circuit fluidique considéré, par exemple une bulle d'air 340. Les petits volumes de ce deuxième composé fusible sont déposés par exemple à la pipette dans des emplacements prévus sur le bord du canal considéré. L'emplacement de la bulle d'air 340 entre les deux corps 350 de composés fusibles dans chaque canal est aussi prévu dans le circuit micro-fluidique. Ces emplacements peuvent être obtenus par exemple par usinage au moyen d'une fraise, ou par gravure laser, ou encore par moulage du circuit.

La température de fusion du deuxième composé fusible est choisie pour être supérieure à la valeur de température T1 employée pour activer le module d'élution M2 décrit ci-dessus, et aussi supérieure ou égale à la valeur de température T2 appliquée au dispositif 31 de contrôle d'accès fluidique et à la chambre 30. Le deuxième composé fusible peut être également une paraffine avec une température de fusion de 55°C (la paraffine choisie est par exemple la tetracosane). Le deuxième composé fusible est choisi avec une température de fusion supérieure à celle du premier composé fusible 25 utilisé dans le module d'élution M2, ceci afin de ne pas fondre lors de l'activation du module d'élution M2.

Le principe de fonctionnement de ce dispositif 310 de scellement est le suivant :
- Au-dessous de sa température de fusion, donc pendant les étapes d'injection de l'éluant et donc d'élution des particules collectées présentées ci-dessus, les deux corps 350 restent piégés sur le bord du canal : le liquide peut circuler librement (figure 6A - P10).
- Au-dessus de sa température de fusion (à la température T2), le deuxième composé fusible formant les deux corps 350 fond, la bulle d'air 340 sous pression du fait de la chaleur peut se dilater en poussant les deux corps 350 fondus dans le canal. Il n'y a pas de pression dans le canal principal autre que la pression hydrostatique, et les bouchons fondus soumis à la poussée d'Archimède et aux forces capillaires restent liquides durant toute la durée du chauffage nécessaire aux analyses (par exemple 30 minutes). Expérimentalement, on observe ainsi une légère remontée des deux corps fondus dans le canal (la paraffine étant moins dense que l'eau), la géométrie de la vanne favorisant cette remontée (figure 6A - P20) dans le canal 360.
- Lors du refroidissement (à la température T3), cette légère remontée des corps 350 est suffisante pour que lors de la contraction de la bulle d'air 340, du liquide venant de la chambre 30 de réaction vienne remplir l'espace disponible, laissant une bonne partie du deuxième composé fusible se solidifier dans le canal (figure 6A - P30).

A haute température, c'est-à-dire au-dessus de la température de fusion T2, en surnageant dans les canaux 360 au-dessus de la chambre 30 de réaction, les corps 350 présents à l'état fondu contribuent pour leur part à limiter l'évaporation du liquide.

A basse température, c'est-à-dire en-dessous de la température de fusion T2, après le chauffage de la chambre 30 ou encore le cycle de chauffe, le deuxième composé fusible revient à l'état solide et les corps 350 scellent complètement les différents canaux, isolant les éventuels amplicons du milieu extérieur. De telles vannes qui isolent la chambre de réaction 30 par un retour à la température ambiante permettent de garantir leur scellement en toute circonstance que ce soit une erreur de manipulation ou encore un dysfonctionnement de l'instrument.

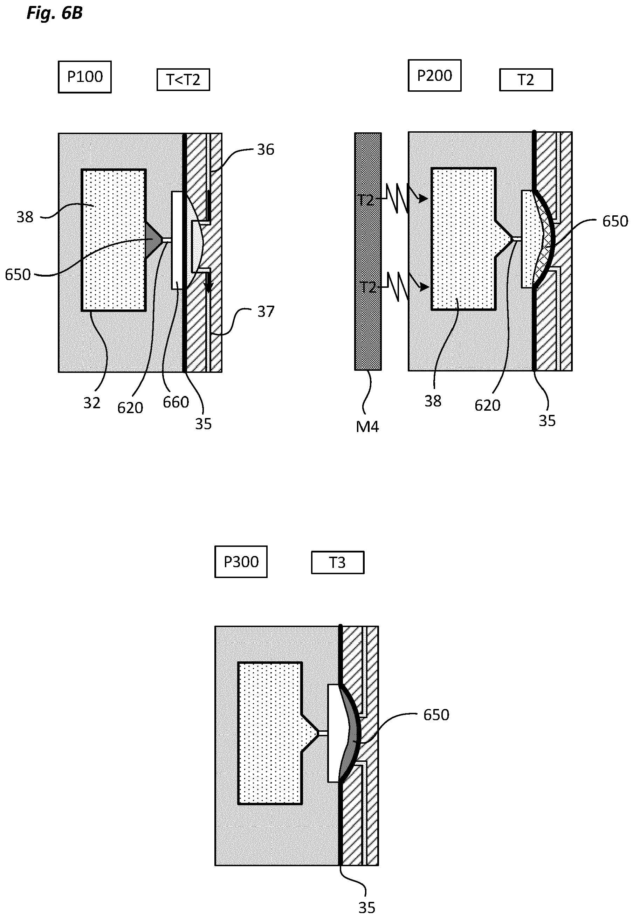

En référence à la figure 6B, la deuxième variante du dispositif de scellement est la suivante.

Ce dispositif de scellement présente la particularité de s'intégrer au dispositif 31 d'isolation déjà décrit ci-dessus et vient ainsi compléter ce dispositif 31 d'isolation déjà décrit et lui ajouter la fonction de scellement.

Le dispositif de scellement comporte ainsi un canal fluidique 620 débouchant d'un côté dans l'espace interne du réservoir 32, rempli du volume d'air 38, et de l'autre côté dans un espace 660 fermé par la membrane 35. Le dispositif comporte également initialement un corps 650 réalisé dans un composé fusible obturant initialement ce canal à son extrémité débouchant dans le réservoir 32.

En référence à la figure 6B, le principe de fonctionnement est le suivant :
- Initialement, à la température ambiante, c'est-à-dire au-dessous de la température T2 de fusion du composé fusible utilisé, le corps 650 obture le canal, et en l'absence de chaleur, la membrane 35 est initialement dans sa position d'ouverture, de sorte que le fluide peut circuler librement entre les deux canaux 36, 37 (figure 6B - P100).
- Le module de chauffage M4 est activé, pour chauffer le volume d'air 38 à la température T2, permettant la dilatation du volume d'air ainsi que la fusion du corps 650 du composé fusible. La dilatation du volume d'air permet de pousser le corps de composé fusible fondu vers l'espace 660, le corps venant alors se déposer sur la membrane 35 déformée (figure 6B - P200). La dilatation du volume d'air déforme également la membrane 35 vers sa position de fermeture, obturant le passage entre les deux canaux 36, 37. Par sa dilatation, le volume d'air 38 peut avoir tendance à plaquer le composé fusible contre la surface de la membrane.
- Après refroidissement, à la température T3 inférieure à la température T2, le composé fusible se durcit et vient bloquer et maintenir la membrane 35 dans sa position de fermeture, permettant le scellement de la chambre 30 de réaction (Figure 6B - P300).

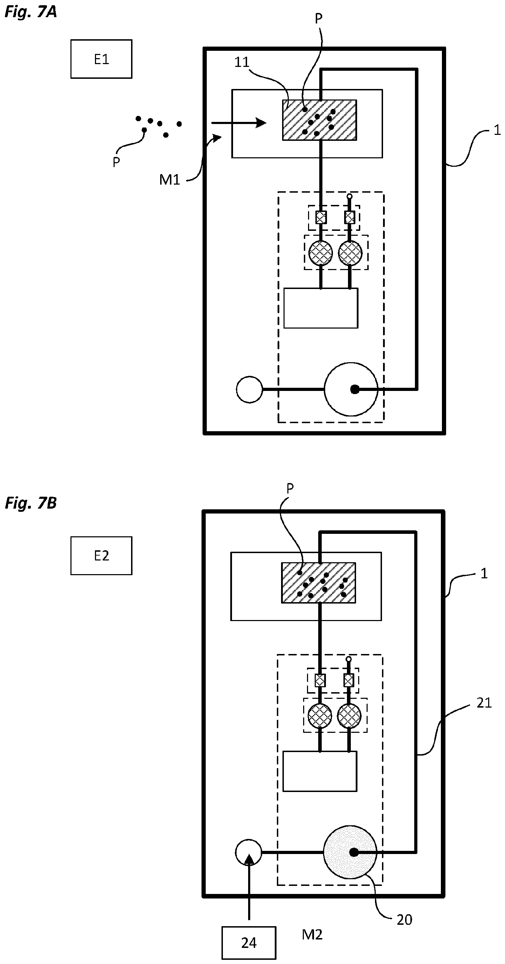

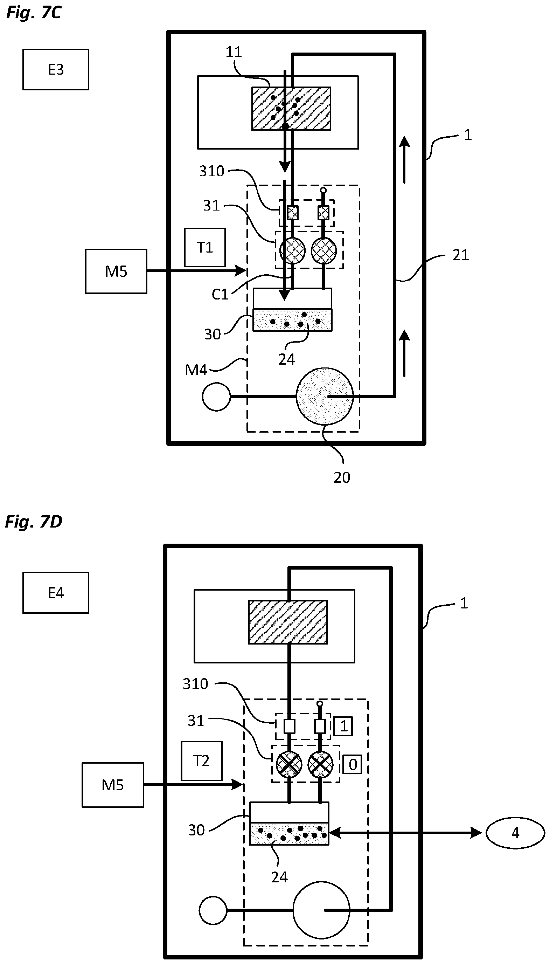

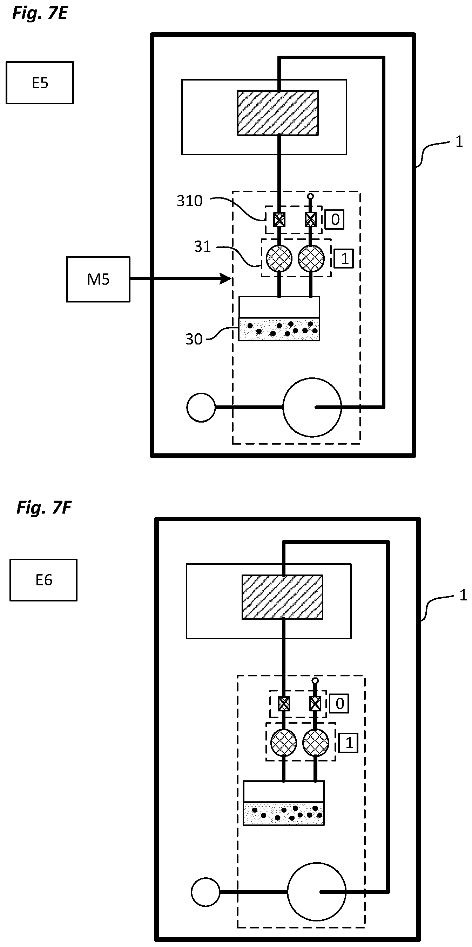

En liaison avec les figures 7A à 7F, on peut définir ci-dessous les différentes étapes de fonctionnement d'un système utilisant le dispositif 31 d'isolation et la première variante du dispositif 310 de scellement :
- E1 - Figure 7A : Le réservoir 20 d'éluant est mis sous-pression avec le liquide d'élution 24 et maintenu sous-pression par le bouchon formé par le premier composé fusible 25. Ce réservoir 20 peut notamment être prérempli dans le composant. Le pré remplissage peut être réalisé en usine et le composant ensuite conservé à une température de stockage adaptée. Les utilisateurs disposent ainsi d'un composant prêt à l'emploi.
- E2 - Figure 7B : Collecte des particules aéroportées au niveau du module M1 de collecte. Les particules P aéroportées sont attirées vers l'électrode de collecte et sont par exemple piégées sur une membrane 11. Cette étape est optionnelle car la collecte des particules pourrait être réalisée en dehors du composant et l'échantillon placé dans le module M1 réalisé dans le composant 1.
- E3 - Figure 7C : Activation du module M4 de chauffage pour atteindre une température ayant une première valeur T1 qui est supérieure à la température de fusion du premier composé fusible 25 fermant initialement le réservoir d'éluant, mais inférieure à la température de fusion des corps 350 employés dans chaque vanne 330a, 330b de scellement à froid de la chambre 30 de réaction. Le module M5 de contrôle active donc le module M4 de chauffage. Le réservoir 20 étant initialement sous pression, le liquide d'élution 24 est libéré du réservoir à travers le canal 21 fluidique d'élution pour atteindre le module de collecte M1 et détacher les particules collées sur la membrane 11. Le liquide d'élution 24, comprenant les particules P, circule dans le circuit fluidique d'élution C1 pour atteindre la chambre 30 de réaction. Le dispositif 31 d'isolation et le dispositif 310 de scellement sont ouverts, permettant au liquide 24 de gagner la chambre 30 de réaction.
- E4 - Figure 7D : On met en oeuvre la réaction de détection dans la chambre 30 de réaction. Classiquement, la réaction nécessite un chauffage de la chambre. Le module M5 de contrôle active donc le module M4 de chauffage. Le module M4 de chauffage est activé, permettant de chauffer la chambre de réaction à une deuxième valeur T2 de température nécessaire à la mise en oeuvre de la réaction biochimique. Cette deuxième valeur de température est supérieure à la première valeur T1 de température. Comme indiqué ci-dessus, le chauffage à la deuxième valeur de température est suffisant pour entraîner :
   ∘ La dilatation de l'air présent dans le réservoir 32 du premier dispositif 31 de contrôle d'accès pour actionner les deux vannes 33a, 33b d'isolation à chaud de la chambre 30 de réaction et isoler la chambre pendant la réaction (vannes à l'état 0) ;
   ∘ La dilatation de la bulle d'air 340 utilisée dans les deux vannes 330a, 330b ;
   ∘ La fusion du deuxième composé fusible utilisé dans les deux vannes 330a, 330b de scellement à froid du deuxième dispositif 310 de contrôle d'accès (vannes à l'état 1) ;
      La chambre 30 étant isolée à chaud par la fermeture des deux vannes d'isolation du premier dispositif 31 de contrôle d'accès fluidique, la réaction peut être mise en oeuvre dans la chambre 30. La température nécessaire à la réaction peut être identique à celle nécessaire pour l'isolation de la chambre, permettant de combiner les deux effets : réaction et isolation de la chambre par une unique activation du module M4 de chauffage et une même consigne pour atteindre la température souhaitée. La réaction d'amplification, par exemple de type PCR ou équivalent, peut utiliser des moyens 4 capables de détecter un signal optique de fluorescence ou d'électro-chimiluminescence ou un changement de couleur ou encore la formation de cristaux à travers une paroi ou les parois de la chambre de réaction, ou un signal électrochimique en vue d'effectuer une détection de matériel biologique dans la chambre 30.
- E5 - Figure 7E : Une fois la réaction terminée, le module M4 de chauffage est désactivé ou commandé à une température inférieure à ladite valeur T2 de température. Cela entraîne la libération de la membrane 35 de chaque vanne 33 d'isolation à chaud (vanne à l'état 1) et le refroidissement du deuxième composé fusible utilisé pour les vannes 330a, 330b de scellement à froid du deuxième dispositif 310 de contrôle d'accès fluidique. Le refroidissement se produit jusqu'à une température ayant une valeur inférieure à ladite valeur T2 de température. Les corps 350 des vannes de scellement à froid se figent et viennent obturer les canaux des deux circuits fluidiques, réalisant le scellement de la chambre 30 de réaction (vannes à l'état 0). Le scellement automatique des chambres 30 par solidification d'un composé dans les canaux reliant la chambre 30 à l'extérieur permet de retirer le composant 1 de l'instrument qui le commandait. La mesure des signaux (optiques ou électrochimiques) émis par les produits de réactions peut être menée en parallèle.
- E6 - Figure 7F : Le composant 1 est scellé de manière étanche, permettant sa manipulation son évacuation sans risque de polluer l'instrument et l'environnement. On remarquera que le fonctionnement passif des vannes de scellement garanti qu'en cas d'erreur de manipulation ou de panne de l'instrument qui , les chambres de réaction seraient scellés. La présence de matériel biologique peut imposer la prise en charge dans une filière d'évacuation spécifique.

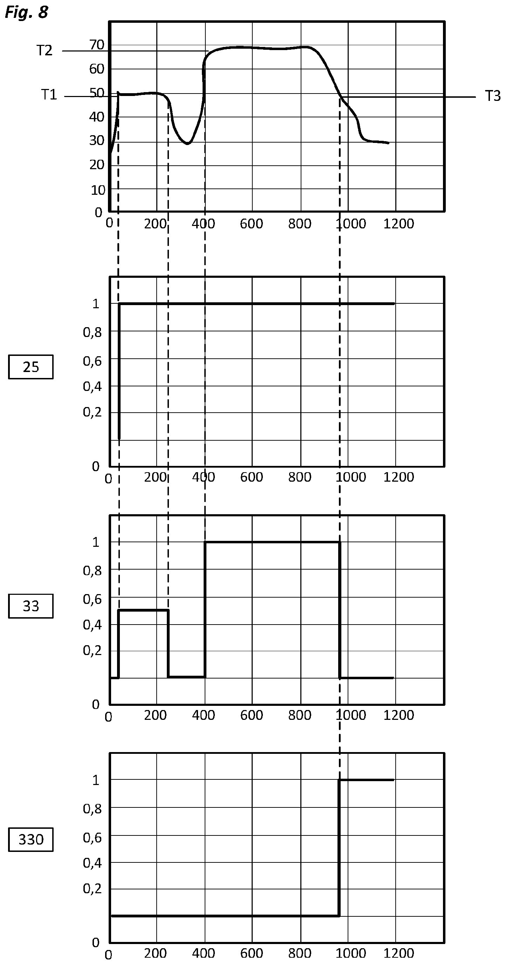

La figure 8 illustre le principe de pilotage des vannes par le cyclage en température appliqué au composant.

Sur cette figure 8, on peut ainsi voir :
- Une première montée en température jusqu'à un premier palier à une première valeur T1 (environ égale à 48°C), entraînant la fusion du premier composé fusible 25 au niveau du module d'élution et la libération du liquide d'élution. La température est trop basse pour entraîner la fermeture des vannes 33 et 330.
- Le module M4 de chauffage est ensuite désactivé ou commandé pour une baisse de la température, permettant un refroidissement du composant ;
- Le module M4 de chauffage est ensuite commandé pour monter jusqu'à un deuxième palier de température, ayant une valeur T2 (environ égale à 70°C pour une réaction d'amplification réalisée par LAMP) qui est supérieure à celle du premier palier. A ce palier de température, les membranes des deux vannes 33 d'isolation à chaud se ferment, permettant une isolation de la chambre 30 de réaction et le deuxième composé fusible fond, permettant ainsi d'amorcer les deux vannes de scellement à froid. Le deuxième palier de température correspond à celui utilisé pour la mise en oeuvre de la réaction de détection dans la chambre 30. Il est maintenu suffisamment longtemps pour terminer cette réaction.
- Après ce deuxième palier, le module M4 de chauffage est désactivé ou régulé à une valeur plus faible T3, entraînant le refroidissement du composant 1 et le figeage des corps 350 réalisés à partir du deuxième composé fusible, pour que ceux-ci viennent obturer les deux circuits fluidiques C1, C2 et sceller la chambre 30 de réaction à froid, par fermeture des deux vannes 330.

Le principe de fonctionnement est identique avec la deuxième variante de réalisation du dispositif 610 de scellement illustré sur la figure 6B.

De manière non limitative, le composant 1 peut être réalisé sous la forme d'une carte micro-fluidique formé d'un empilement de plusieurs couches. L'empilement peut notamment comporter trois substrats réalisés chacun dans un matériau de type COP/COC (Cyclo Oléfin Polymère/Cyclo Olefin Copolymère), polycarbonate ou de type PMMA (Poly-méthacrylate de Méthyle). Il peut notamment présenter des caractéristiques de transparence, suffisantes pour une lecture optique lorsque l'analyse est mise en oeuvre directement dans le composant. Une membrane commune aux différents modules du composant peut être intercalée entre les deux substrats. La membrane est formée d'un matériau très déformable élastiquement, lui permettant de revenir à sa forme initiale après déformation. A titre d'exemple, la membrane peut notamment être réalisée dans des matériaux tels que les élastomères de la famille des silicones tels que les MQ (Methyl-Polysiloxanes), les VMQ (Vinyl-Methyl-Polysiloxanes), les PVMQ (Phenyl-Vynil-Methyl-Polysiloxanes) ou les élastomères de type thermoplastiques (TPE), par exemple les TPE-S, TPS, TPE-E, TPC. Elle joue ainsi le rôle de la membrane déformable poussant le liquide d'élution au niveau du module d'élution et des membranes utilisées dans les deux vannes d'isolation à chaud du dispositif 31 d'isolation.

Les différents circuits fluidiques du composant 1 ainsi que le réservoir 20 d'éluant, le réservoir 32 d'air du premier dispositif 31 de contrôle d'accès et la chambre 30 de réaction, peuvent être réalisés par un usinage ou autre procédé appliqué sur un et/ou les des deux substrats du composant.

Le module M4 de chauffage de l'instrument, en contact avec le composant 1, peut être composé par exemple d'une seule résistance chauffante ou d'un élément chauffant dont la température peut être facilement ajustée comme un module à effet Peltier. Le module M4 de chauffage est avantageusement commun à plusieurs modules du composant, notamment :
- Le module M2 d'élution pour faire fondre le premier composé fusible fermant initialement le réservoir d'éluant ;
- Le module M3 d'analyse pour :
   ∘ Actionner les membranes 35 des deux vannes 33 d'isolation à chaud, par dilatation de l'air stocké dans le réservoir 32 ;
   ∘ Dilater la bulle d'air 340 employée dans chaque vanne 330 de scellement à froid ;
   ∘ Faire fondre le deuxième composé fusible employé dans les deux vannes 330 de scellement à froid ;
   ∘ Assurer la mise en oeuvre de la réaction prévue (par exemple réaction d'amplification) dans la chambre 30 de réaction ;

Un système de régulation de la température peut être employé pour gérer la température de chauffage du module de chauffage. Ce système peut comporter au moins un capteur de température et une boucle de régulation exécutée par le module de contrôle.

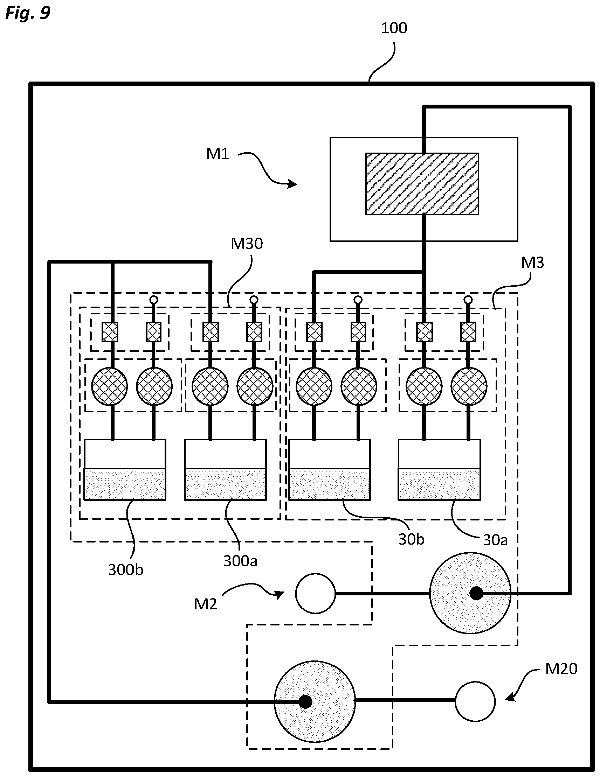

Dans une réalisation particulière du composant 100, représentée sur la figure 9, le module M3 d'analyse du composant 100 peut comporter deux chambres 30a, 30b de réaction en parallèle. En outre, le composant 1 peut également comporter un module M6 de contrôle de la réaction qui comporte un deuxième module M20 d'élution et un deuxième module M30 d'analyse, le module M20 d'élution ayant une architecture identique à celui décrit ci-dessus, à l'exception du fait que son circuit fluidique ne débouche pas dans le module M1 de collecte mais directement dans deux chambres 300a, 300b de contrôle en parallèle du deuxième module M30 d'analyse. Le deuxième module M30 d'analyse a une architecture identique à celui décrit ci-dessus et reçoit le liquide d'élution vierge de toute particule de façon à réaliser des réactions de contrôle. Le premier module M2 d'élution est utilisé pour réaliser l'élution des particules capturées sur la membrane 11, les particules P étant acheminées dans deux chambres parallèles du premier module M3 d'analyse. Le second module d'élution M20 sert à injecter directement l'éluant dans les deux chambres 300a, 300b de contrôle. Ces chambres ont pour but de vérifier le bon déroulement de l'analyse. L'une de ces chambres contient une quantité définie de molécule à détecter (contrôle positif) et donnera, si le circuit d'élution est fonctionnel et si les réactifs et les éléments biologiques sont intacts, un signal positif. L'autre chambre sert de contrôle négatif et vise notamment à vérifier la non pollution du système, la bonne calibration du dispositif de mesure. Les deux modules M3, M30 d'analyse (celui pour l'échantillon et celui pour les contrôles) étant sur un unique module de chauffage et la température étant l'unique variable influençant l'élution, les contrôles sont dans ce cas des bons indicateurs d'une élution et d'une analyse correctes.

Le composant de collecte et d'analyse des particules aéroportées dispose de plusieurs avantages, parmi lesquels :
- La possibilité de réunir tous les modules dans un même composant, permettant de fabriquer un dispositif simple à manipuler, parfaitement autonome et facilement déployable ;
- La possibilité de piloter toutes les étapes sans raccordements physiques à réaliser tels que des raccords pneumatiques, des liaisons mécaniques, etc. Le composant 1 est inséré dans un instrument qui commande le module de collecte M1 au moyen de deux contacts électriques et les modules M2 et M3 au moyen d'une surface chauffante ;
- La possibilité de n'utiliser qu'un seul module de chauffage M4, commun à tout le composant pour réaliser toutes les étapes du procédé d'injection de réactifs, de transfert d'échantillon et d'analyse ;
- Un composant fluidique simple pouvant être réalisé sous la forme d'une carte facile à manipuler ;
- La possibilité pour un opérateur d'enchainer aisément plusieurs collectes et analyses sans devoir décontaminer le collecteur, des pièces de jonction entre le collecteur et le module de préparation d'échantillon et le module d'analyse. L'instrument commandant le composant 1 n'étant pas en contact avec les échantillons et les réactifs biologique, il est immédiatement disponible pour de nouvelles collectes et analyses ;
La solution peut notamment se baser en grande partie sur l'utilisation de différentes paraffines (alcanes linéaires) fondant à des températures différentes et précises comprises par exemple entre (40 et 70°C), par exemple le docosane (42-45°C), le tetracosane (49-52°C) ou le Dotriacontane (65-70°C).

## Revendications

1. Procédé de récupération et d'analyse de particules aéroportées, comprenant :
- Une étape d'élution de particules (P) collectées sur une surface de collecte, par injection d'un liquide d'élution (24) à travers un circuit fluidique d'élution (C1), ledit circuit fluidique d'élution étant agencé pour acheminer lesdites particules (P) collectées à destination d'une chambre de réaction d'un module d'analyse,
- Une étape d'analyse des particules aéroportées récupérées dans la chambre (30) de réaction,
- **Caractérisé en ce que** :
- L'étape d'élution est mise en oeuvre par chauffage d'un premier réservoir (20) contenant ledit liquide d'élution à une première valeur (T1) de température pour faire fondre un premier composé fusible (25) maintenant ledit premier réservoir (20) sous pression,
- L'étape d'analyse est mise en oeuvre par chauffage de la chambre (30) de réaction à une deuxième valeur (T2) de température, supérieure à la première valeur (T1) de température, pour :
∘ Activer une réaction de détection dans ladite chambre (30) de réaction en vue de réaliser une analyse des particules aéroportées,
∘ Isoler ladite chambre (30) de réaction pendant la réaction de détection par activation d'un dispositif (31) d'isolation de la chambre (30) de réaction,
∘ Amorcer un dispositif (310) de scellement de la chambre de réaction, par fusion d'un deuxième composé fusible,
- Une étape de scellement de ladite chambre (30) de réaction par figeage du deuxième composé fusible lorsque la température descend à une troisième valeur (T3) inférieure à ladite deuxième valeur (T2).

2. Procédé selon la revendication 1, **caractérisé en ce qu'**il comporte une étape de contrôle de la réaction par injection d'un deuxième liquide d'élution dans au moins une chambre de contrôle identique à ladite chambre de réaction.

3. Procédé selon la revendication 1 ou 2, **caractérisé en ce que** chaque liquide d'élution embarque des réactifs utilisés lors de la réaction dans la chambre (30) de réaction.

4. Procédé selon l'une des revendications 1 à 3, **caractérisé en ce que** la réaction est de type amplification biomoléculaire.

5. Procédé selon l'une des revendications 1 à 4, **caractérisé en ce que** l'activation du dispositif (31) d'isolation de la chambre (30) de réaction consiste à fermer une première vanne (33a) d'isolation agencée sur le circuit fluidique d'élution (C1) et dotée d'une membrane (35) déformable.

6. Procédé selon la revendication 5, **caractérisé en ce que** l'amorçage du dispositif (310) de scellement de la chambre (30) de réaction consiste à libérer le deuxième composé fusible dans le circuit fluidique d'élution (C1).

7. Procédé selon la revendication 5, **caractérisé en ce que** l'amorçage du dispositif de scellement de la chambre (30) de réaction consiste à libérer le deuxième composé fusible pour que celui-ci vienne se déposer sur la membrane déformable de la première vanne (33a) d'isolation.

8. Dispositif de récupération et d'analyse de particules aéroportées adapté pour mettre en oeuvre le procédé tel que défini dans l'une des revendications 1 à 7, comprenant :
- Un module (M3) d'analyse de particules aéroportées comportant une chambre (30) de réaction,
- Un module (M2) d'élution de particules (P) collectées sur une surface de collecte, par injection d'un liquide d'élution (24) à travers un circuit fluidique d'élution (C1), ledit circuit fluidique d'élution (C1) étant agencé pour acheminer lesdites particules (P) collectées à destination de la chambre de réaction du module d'analyse, **Caractérisé en ce que** :
- Le module (M2) d'élution comporte un premier réservoir (20) contenant le liquide d'élution, ledit premier réservoir (20) étant maintenu sous pression par un premier composé fusible (25), ledit premier composé fusible étant choisi pour fondre par un premier chauffage à une première valeur (T1) de température en vue de libérer ledit liquide d'élution (20),
- Le module (M3) d'analyse comporte :
∘ Un dispositif (31) d'isolation de la chambre (30) de réaction, configuré pour isoler ladite chambre de réaction lors d'un deuxième chauffage de la chambre (30) de réaction à une deuxième valeur (T2) de température supérieure à ladite première valeur (T1) de température,
∘ Un dispositif (310, 610) de scellement de la chambre de réaction, amorcé par fusion d'un deuxième composé fusible configuré pour figer à une troisième valeur (T3) inférieure à ladite deuxième valeur (T2).

9. Dispositif selon la revendication 8, **caractérisé en ce qu'**il comporte un module de contrôle de la réaction comportant au moins une chambre de contrôle identique à ladite chambre de réaction.

10. Dispositif selon la revendication 8 ou 9, **caractérisé en ce que** le liquide d'élution embarque des réactifs utilisés lors de la réaction dans la chambre (30) de réaction.

11. Dispositif selon l'une des revendications 8 à 10, **caractérisé en ce que** le dispositif (31) d'isolation de la chambre (30) de réaction comporte une première vanne (33a) d'isolation agencée sur le circuit fluidique d'élution (C1) et dotée d'une membrane (35) déformable.

12. Dispositif selon la revendication 11, **caractérisé en ce que** le dispositif (310) de scellement de la chambre (30) de réaction comporte un corps (350) du deuxième composé fusible positionné pour se libérer dans le circuit fluidique d'élution lors de l'amorçage.

13. Dispositif selon la revendication 11, **caractérisé en ce que** le dispositif (310) de scellement de la chambre (30) de réaction comporte un corps (650) du deuxième composé fusible positionné pour se déposer sur la membrane (35) déformable lors de l'amorçage.

14. Dispositif selon l'une des revendications 8 à 13, **caractérisé en ce qu'**il comporte un composant (1) de collecte et d'analyse de particules aéroportées réalisé sous la forme d'un élément monobloc, comportant
- Un module (M1) de collecte des particules aéroportées ;
- Le module (M2) d'élution des particules aéroportées collectées ;
- Le module (M3) d'analyse des particules éluées vers la chambre (30) de réaction ;

15. Dispositif selon la revendication 14, **caractérisé en ce qu'**il comporte un module (M4) de chauffage réalisé sous la forme d'un instrument sur lequel ledit composant (1) de collecte et d'analyse vient s'adapter de manière amovible.

## Patentansprüche

1. Verfahren zur Rückgewinnung und Analyse von luftgetragenen Partikeln, das Folgendes beinhaltet:
- einen Schritt des Eluierens von Partikeln (P), die auf einer Sammeloberfläche gesammelt werden, durch Einspritzen einer Elutionsflüssigkeit (24) durch einen Elutionsfluidkreislauf (C1), wobei der Elutionsfluidkreislauf dazu eingerichtet ist, die gesammelten Partikel (P) in Richtung einer Reaktionskammer eines Analysemoduls zu führen,
- einen Schritt des Analysierens der zurückgewonnenen luftgetragenen Partikel in der Reaktionskammer (30),
- **dadurch gekennzeichnet, dass**:
- der Schritt des Eluierens durch Erhitzen eines ersten Behälters (20), der die Elutionsflüssigkeit enthält, auf einen ersten Temperaturwert (T1) umgesetzt wird, um eine erste schmelzbare Zusammensetzung (25), die den ersten Behälter (20) unter Druck hält, zum Schmelzen zu bringen,
- der Schritt des Analysierens durch Erhitzen der Reaktionskammer (30) auf einen zweiten Temperaturwert (T2), der größer als der erste Temperaturwert (T1) ist, umgesetzt wird, um:
∘ eine Nachweisreaktion in der Reaktionskammer (30) zu aktivieren, um eine Analyse der luftgetragenen Partikel durchzuführen,
∘ die Reaktionskammer (30) während der Nachweisreaktion zu isolieren, indem eine Isoliervorrichtung (31) der Reaktionskammer (30) aktiviert wird,
∘ eine Verschließvorrichtung (310) der Reaktionskammer auszulösen, indem eine zweite schmelzbare Zusammensetzung zum Schmelzen gebracht wird,
- einen Schritt des Verschließens der Reaktionskammer (30), indem die zweite schmelzbare Zusammensetzung zum Erstarren gebracht wird, wenn die Temperatur auf einen dritten Wert (T3), der kleiner als der zweite Wert (T2) ist, absinkt.

2. Verfahren nach Anspruch 1, **dadurch gekennzeichnet, dass** es einen Schritt des Kontrollierens der Reaktion umfasst, indem eine zweite Elutionsflüssigkeit in mindestens eine Kontrollkammer, die zu der Reaktionskammer identisch ist, eingespritzt wird.

3. Verfahren nach Anspruch 1 oder 2, **dadurch gekennzeichnet, dass** jede Elutionsflüssigkeit Reagenzien enthält, die während der Reaktion in der Reaktionskammer (30) verwendet werden.

4. Verfahren nach einem der Ansprüche 1 bis 3, **dadurch gekennzeichnet, dass** die Reaktion vom Typ biomolekulare Amplifikation ist.

5. Verfahren nach einem der Ansprüche 1 bis 4, **dadurch gekennzeichnet, dass** das Aktivieren der Isoliervorrichtung (31) der Reaktionskammer (30) darin besteht, ein erstes Isolierventil (33a) zu schließen, das in dem Elutionsfluidkreislauf (C1) eingerichtet ist und über eine verformbare Membran (35) verfügt.

6. Verfahren nach Anspruch 5, **dadurch gekennzeichnet, dass** das Auslösen der Verschließvorrichtung (310) der Reaktionskammer (30) darin besteht, die zweite schmelzbare Zusammensetzung in dem Elutionsfluidkreislauf (C1) freizusetzen.

7. Verfahren nach Anspruch 5, **dadurch gekennzeichnet, dass** das Auslösen der Verschließvorrichtung der Reaktionskammer (30) darin besteht, die zweite schmelzbare Zusammensetzung freizusetzen, damit sich diese an der verformbaren Membran des ersten Isolierventils (33a) ablagert.

8. Vorrichtung zur Rückgewinnung und Analyse von luftgetragenen Partikeln, die zur Umsetzung des Verfahrens nach einem der Ansprüche 1 bis 7 angepasst ist und Folgendes beinhaltet:
- ein Modul (M3) zum Analysieren von luftgetragenen Partikeln, das eine Reaktionskammer (30) umfasst,
- ein Modul (M2) zum Eluieren von Partikeln (P), die auf einer Sammeloberfläche gesammelt werden, durch Einspritzen einer Elutionsflüssigkeit (24) durch einen Elutionsfluidkreislauf (C1), wobei der Elutionsfluidkreislauf (C1) dazu eingerichtet ist, die gesammelten Partikel (P) in Richtung der Reaktionskammer des Analysemoduls zu führen,
**dadurch gekennzeichnet, dass**:
- das Elutionsmodul (M2) einen ersten Behälter (20) umfasst, der die Elutionsflüssigkeit enthält, wobei der erste Behälter (20) durch eine erste schmelzbare Zusammensetzung (25) unter Druck gehalten wird, wobei die erste schmelzbare Zusammensetzung so ausgewählt ist, dass sie durch ein erstes Erhitzen auf einen ersten Temperaturwert (T1) schmilzt, um die Elutionsflüssigkeit (20) freizusetzen,
- wobei das Analysemodul (M3) Folgendes umfasst:
∘ eine Isoliervorrichtung (31) der Reaktionskammer (30), die dazu konfiguriert ist, die Reaktionskammer während eines zweiten Erhitzens der Reaktionskammer (30) auf einen zweiten Temperaturwert (T2), der größer als der erste Temperaturwert (T1) ist, zu isolieren,
∘ eine Verschließvorrichtung (310, 610) der Reaktionskammer, die durch das Schmelzen einer zweiten schmelzbaren Zusammensetzung ausgelöst wird, welche dazu konfiguriert ist, bei einem dritten Wert (T3), der kleiner als der zweite Wert (T2) ist, zu erstarren.

9. Vorrichtung nach Anspruch 8, **dadurch gekennzeichnet, dass** sie ein Modul zum Kontrollieren der Reaktion umfasst, das mindestens eine Kontrollkammer, die zu der Reaktionskammer identisch ist, umfasst.

10. Vorrichtung nach Anspruch 8 oder 9, **dadurch gekennzeichnet, dass** die Elutionsflüssigkeit Reagenzien enthält, die während der Reaktion in der Reaktionskammer (30) verwendet werden.

11. Vorrichtung nach einem der Ansprüche 8 bis 10, **dadurch gekennzeichnet, dass** die Isoliervorrichtung (31) der Reaktionskammer (30) ein erstes Isolierventil (33a) umfasst, das in dem Elutionsfluidkreislauf (C1) eingerichtet ist und über eine verformbare Membran (35) verfügt.

12. Vorrichtung nach Anspruch 11, **dadurch gekennzeichnet, dass** die Verschließvorrichtung (310) der Reaktionskammer (30) einen Körper (350) der zweiten schmelzbaren Zusammensetzung umfasst, der positioniert ist, um während des Auslösens in dem Elutionsfluidkreislauf freigesetzt zu werden.

13. Vorrichtung nach Anspruch 11, **dadurch gekennzeichnet, dass** die Verschließvorrichtung (310) der Reaktionskammer (30) einen Körper (650) der zweiten schmelzbaren Zusammensetzung umfasst, der positioniert ist, um sich während des Auslösens an der verformbaren Membran (35) abzulagern.

14. Vorrichtung nach einem der Ansprüche 8 bis 13, **dadurch gekennzeichnet, dass** sie eine Komponente (1) zum Sammeln und Analysieren von luftgetragenen Partikeln umfasst, die in Form eines einteiligen Elements ausgebildet ist und Folgendes umfasst:
- ein Modul (M1) zum Sammeln der luftgetragenen Partikel;
- das Modul (M2) zum Eluieren der gesammelten luftgetragenen Partikel;
- das Modul (M3) zum Analysieren der zu der Reaktionskammer (30) eluierten Partikel.

15. Vorrichtung nach Anspruch 14, **dadurch gekennzeichnet, dass** sie ein Heizmodul (M4) umfasst, das in Form eines Instruments ausgebildet ist, an das sich die Sammel- und Analysekomponente (1) entfernbar anpasst.

## Claims

1. Method for collecting and analysing airborne particles, comprising:
- a step of eluting particles (P) precipitated on a collecting surface, by injecting an elution liquid (24) through an elution fluidic circuit (C1), said elution fluidic circuit being arranged to convey said precipitated particles (P) to a reaction chamber of an analysis module,
- a step of analysing the airborne particles collected in the reaction chamber (30),
- **characterized in that**:
- the eluting step is carried out by heating a first reservoir (20) containing said elution liquid to a first temperature value (T1) in order to make a first meltable compound (25) melt, maintaining said first reservoir (20) under pressure,
- the analysing step is carried out by heating the reaction chamber (30) to a second temperature value (T2) higher than the first temperature value (T1), so as to:
∘ activate a detection reaction in said reaction chamber (30) with a view to carrying out an analysis of the airborne particles,
∘ isolate said reaction chamber (30) during the detection reaction by activating a device (31) for isolating the reaction chamber (30),
∘ initiate a device (310) for sealing the reaction chamber, by melting a second meltable compound,
- a step of sealing said reaction chamber (30) by setting the second meltable compound, which occurs when the temperature drops to a third value (T3) lower than said second value (T2).

2. Method according to Claim 1, **characterized in that** it comprises a step of controlling the reaction by injecting a second elution liquid into at least one control chamber identical to said reaction chamber.

3. Method according to Claim 1 or 2, **characterized in that** each elution liquid contains reagents used in the reaction in the reaction chamber (30).

4. Method according to one of Claims 1 to 3, **characterized in that** the reaction is a biomolecular-amplification reaction.

5. Method according to one of Claims 1 to 4, **characterized in that** activation of the device (31) for isolating the reaction chamber (30) consists in closing a first isolation valve (33a) arranged in the elution fluidic circuit (C1) and equipped with a deformable membrane (35).

6. Method according to Claim 5, **characterized in that** initiation of the device (310) for sealing the reaction chamber (30) consists in releasing the second meltable compound into the elution fluidic circuit (C1).

7. Method according to Claim 5, **characterized in that** initiation of the device for sealing the reaction chamber (30) consists in releasing the second meltable compound so that the latter deposits on the deformable membrane of the first isolation valve (33a).

8. Device for collecting and analysing airborne particles and suitable for implementing the method such as defined in one of Claims 1 to 7, comprising:
- a module (M3) for analysing airborne particles comprising a reaction chamber (30),
- a module (M2) for eluting particles (P) precipitated on a collecting surface, by injecting an elution liquid (24) through an elution fluidic circuit (C1), said elution fluidic circuit (C1) being arranged to convey said precipitated particles (P) to the reaction chamber of the analysis module,
**characterized in that**:
- the eluting module (M2) comprises a first reservoir (20) containing the elution liquid, said first reservoir (20) being maintained under pressure by a first meltable compound (25), said first meltable compound being chosen for its ability to melt as a result of a first operation of heating to a first temperature value (T1), with a view to releasing said elution liquid (20),
- the analysing module (M3) comprises:
∘ a device (31) for isolating the reaction chamber (30), said device being configured to isolate said reaction chamber on a second operation of heating the reaction chamber (30) to a second temperature value (T2) higher than said first temperature value (T1),
∘ a device (310, 610) for sealing the reaction chamber, said device being initiated by melting a second meltable compound configured to set at a third value (T3) lower than said second value (T2).

9. Device according to Claim 8, **characterized in that** it comprises a reaction-controlling module comprising at least one control chamber identical to said reaction chamber.

10. Device according to Claim 8 or 9, **characterized in that** the elution liquid contains reagents used in the reaction in the reaction chamber (30).

11. Device according to one of Claims 8 to 10, **characterized in that** the device (31) for isolating the reaction chamber (30) comprises a first isolation valve (33a) arranged in the elution fluidic circuit (C1) and equipped with a deformable membrane (35).

12. Device according to Claim 11, **characterized in that** the device (310) for sealing the reaction chamber (30) comprises a body (350) of the second meltable compound positioned to be released into the elution fluidic circuit on initiation.

13. Device according to Claim 11, **characterized in that** the device (310) for sealing the reaction chamber (30) comprises a body (650) of the second meltable compound positioned to deposit on the deformable membrane (35) on initiation.

14. Device according to one of Claims 8 to 13, **characterized in that** it comprises a component (1) for precipitating and analysing airborne particles, said component taking the form of an element of integral construction, comprising:
- a module (M1) for precipitating airborne particles;
- the module (M2) for eluting the precipitated airborne particles;
- the module (M3) for analysing the particles eluted to the reaction chamber (30).

15. Device according to Claim 14, **characterized in that** it comprises a heating module (M4) taking the form of an instrument into which said precipitating and analysing component (1) is removably fitted.
